# EUROPEAN PATENT SPECIFICATION

(11) **EP 2 978 416 B1**
(45) Date of publication and mention of the grant of the patent: **26.10.2016**
(21) Application number: 14713808.5
(22) Date of filing: 25.03.2014
(51) Int. Cl.: A61K 31/01, A61P 1/00

(54) **PHARMACEUTICAL FORMULATION COMPRISING PHOSPHATIDYLCHOLINE FOR THE TREATMENT OF ULCERATIVE COLITIS**
PHARMAZEUTISCHE FORMULIERUNG MIT PHOSPHATIDYLCHOLIN ZUR BEHANDLUNG VON COLITIS ULCEROSA
FORMULATION PHARMACEUTIQUE COMPORTANT DE LA PHOSPHATIDYLCHOLINE POUR LE TRAITEMENT DE LA COLITE ULCÉREUSE

(30) Priority: 26.03.2013 EP 13161089
(43) Date of publication of application: 03.02.2016
(73) Proprietor: Lipid Therapeutics GmbH, 69126 Heidelberg (DE)
(72) Inventor: KEILHAUER, Gerhard, 67125 Dannstadt (DE)
(74) Representative: Jacobi, Markus Alexander
(86) International application number: PCT/EP2014/055947
(87) International publication number: WO 2014/154683

(56) References cited:
- EP-A1- 0 479 121
- EP-A2- 0 112 174

## Description

The present invention relates to pharmaceutical formulations containing a phosphatidylcholine for the treatment of chronic inflammatory diseases, such as ulcerative colitis, and in particular ulcerative colitis in ASA-refractory individuals.

Phosphatidylcholines (PC) are a known class of naturally occurring products. They can be found together with other lipid compounds as one component in lecithins in animal and plant tissues. A typical phosphatidylcholine is composed of the following structural elements: phosphoric acid, choline, fatty acids and glycerol. The composition of the fatty acid residues in a PC has an important influence on the properties of the phosphatidylcholine. The phosphatidylcholines can either be synthesized or extracted chemically or mechanically from natural sources, such as from soy beans, other plants or eggs. Phosphatidylcholines normally have a low solubility in water but can be dispersed in water. They are not particularly stable under acidic conditions.

Various chronic inflammatory diseases, such as ulcerative colitis, affect children and adults with severe consequences. These diseases, such as ulcerative colitis, are characterized by frequent bouts of watery diarrhea, accompanied by blood and/or mucus. Other symptoms include cramping, abdominal pain, pain on opening the bowels, urgent and frequent need to open the bowels, the sensation of incomplete emptying of the bowels, nausea, no appetite, weight loss, tiredness, skin rashes, mouth ulcers, joint pains and anemia. Chronic inflammatory diseases and ulcerative colitis affect more of the colon than the rectum alone. The symptoms vary according to the degree of inflammation in the bowel and whether or not the lining of the bowel has become ulcerated. Chronic ulcerative colitis also can cause changes in the liver, sclerosing cholangitis and can increase the risk of developing bowel cancer.

Several methods for the treatment of chronic inflammatory diseases have been described in the literature, however many methods should not be applied over a long period of time and/or have serious side-effects.

The document WO 1995/18622 describes the use of 2-hydroxy-5-phenyl-azobenzoic acid as chemo-protective substance for the treatment of diseases of the colon. In WO 2000/07577, the use of a phosphatidylcholine for the treatment of diseases of the intestines is described.

US 6,677,319 discloses the use of a phosphatidylcholine containing lecithin-composition for the treatment of colitis, however the phosphatidylcholine product used has a high content of phosphatidylethanolamine (PE) and phosphatidylinositol (PI). Typically known lecithin products can contain 30-70 % by weight of PC but also contain 18-21 % (PE) and 6-8 % of (PI), which often do not or not significantly contribute to the pharmaceutical or clinical activity. Furthermore, the stability of the phosphatidylcholine product used is not satisfying.

WO 2009/015891 and EP-A 2185158 mention the treatment of patients having steroid-dependent ulcerative colitis by using a lecithin product, which however has a high content of phosphatidylethanolamine (PE) and phosphatidylinositol (PI). The phosphatidyl choline-rich phospholipid mixture (Centroplex FP) contains typically not less than 30 % PC, 18-21 % phosphatidylethanolamine, and 6-8 % phosphatidylinositol. Verum containing 43.10 % Centrolex, microcrystalline cellulose, magnesium stearate and hydrated silica and were embedded with Eudragit®.

Retarded release phosphatidylcholine formulations for patients with chronic active ulcerative colitis are described by W. Stremmel in GUT, British Medical Association (Vol. 54, No. 7, 01 July 2005, pages 966-971).These PC-formulations contain significant amounts of other phospholipids and the retarded release formulations lead to some side-effects, such as flatulence. As these side-effects can be observed with a high frequency, new formulations are needed to improve the compliance for patients. The PC rich phospholipid mixture used by W. Stremmel (Sterpur P-30 Granulat; Stem-Lecithin and Soja GmbH, Hamburg, Germany) contained 30 % phosphatidylcholine, 21 % phosphatidylethanolamine and 8 % phosphatidylinositol. Retarded release phosphatidylcholine granules consisted of lecithin (of provider Stem) embedded with Eudragit®, Avicel® PH102 (FMC Company, USA) and Syloid® 244 (silica).

Commercial phosphatidylcholine/lecithin products, such as Sigma P5638, often contain only less than 50 percent by weight of PC and high amounts of e.g. phosphatidylethanolamine (PE) and phosphatidylinositol (PI). Other commercial phosphatidylcholine/lecithin products contain significant amounts of nonpolar lipids and/or phosphatidylethanolamine-derivatives. The phosphatidylcholine product (PC) according to the present invention can be described as "substantially free of phosphatidylinositol (PI) and phosphatidylethanolamine (PE)", corresponding to less than 0.1 % w/w, respectively, of the entire lipid content of the product.

C. Vetter describes various treatment options in inflammatory bowel disease and new aspects for established therapies in European Gastroenterology and Hepatology Review 2011 (Vol. 7, No. 2, May 2011, pages 104-107), however the publication only generally describes a clinical study with a lecithin product without describing the pharmaceutical formulation used.

Furthermore, W. Stremmel discloses phosphatidylcholine for steroid-refractory chronic ulcerative colitis in Annals of Internal Medicine, New York (Vol. 147(9), 06.11.2007), but the phosphatidylcholine product disclosed comprises high amounts of phosphatidylethanolamines and other phospholipids.

Ulcerative colitis is a common chronic inflammatory disease of the bowel in adults and children. It is defined as a diffuse inflammatory condition of the colonic mucosa that might extend from the rectum to the caecum causing erosions of the colon mucosa. During the clinical course of the disease, a proximal extension can be observed in many patients with initially diagnosed proctosigmoiditis. Main manifestations are pancolitis, right sided colitis, left sided colitis, proctosigmoiditis and proctitis. In some cases, an isolated caecal involvement can be observed besides rectal or sigmoid inflammation. Typical symptoms are bloody diarrhoea, pain during bowel movements, spasms, fever, anaemia, and weight loss.

The aetiology of ulcerative colitis is unknown. Based on epidemiological examinations, it is discussed that beside a genetic disposition further factors like environmental influences trigger the disease. A disturbed mucosal barrier seems an initiating factor of the disease and subsequent attacks from colonic commensal bacterial flora result in the inflammation of the mucosa. Normally, mucosal cells are protected against such attacks by a continuous, hydrophobic, and adherent mucus layer. This layer contains phospholipids, which are made up of phosphatidylcholines and lyso-phosphatidyl-choline.

Classical treatment of ulcerative colitis is focused on controlling flare-ups and reducing the chances of further flare-ups or complications. For many years, the drug compound Mesalazine (5-amino-2-hydroxy-benzoic acid) was a standard therapy for mild and moderate ulcerative colitis. Mesalazine and the drug compounds Sulfasalazin, Olsalazine, Balsalazine are also together named 5-ASA-compounds. There is however an increasing number of ASA-refractory individuals. Many individuals become 5-ASA-refractory or do not tolerate 5-ASA-compounds, in particular the compound Mesalazine.

Other treatments for ulcerative colitis and flare-ups include steroid therapy, the use of immune-suppressants, the use of TNF-antagonists and/or surgery. Although steroids are often effective at reducing bouts of inflammation, adverse side effects are associated with long term steroid use. ASA-refractory in the context of this invention means that the symptoms of the disease ulcerative colitis are not considerably improved by using the above mentioned 5-ASA-compounds, and in particular 5-amino-2-hydroxy-benzoic acid.

The present invention relates to the treatment of ulcerative colitis by using specific formulations of particular phosphatidylcholine products. The invention also relates to the new formulations as described in the patent claims.

Phosphatidylcholines are responsible for the establishment of the protective hydrophobic surface, holding a key role in mucosal defence processes. Patients with ulcerative colitis have significantly less phosphatidylcholines in their intestinal mucus compared to Crohn's disease patients and healthy controls. A damaged phosphatidylcholine layer may lead to inflammation and ulceration. Phosphatidylcholines and other lipids were shown in the literature to inhibit pro-inflammatory signal processes and to alleviate inflammatory activity caused by ulcerative colitis. From in vitro and in vivo experiments in mice and rats it can be seen that phosphatidylcholines are actively secreted into the ileum. Phosphatidylcholines are moved continuously from caecum to rectum by colonic activity, the rectum being the last area supplied with phosphatidylcholines.

It is one objective of the invention to provide new pharmaceutical formulations for ulcerative colitis treatment, in particular for ulcerative colitis treatment in patients that are refractory to classical treatment (such as 5-ASA, steroids or immunesuppressants). These formulations have an improved efficacy and/or less severe consequences or side effects than the limited treatment options with known classical formulations available. Furthermore, side effects (such as flatulence) are observed with a lower frequency.

Furthermore, the daily dose of PC in many cases can be kept low, which improves patients' compliance. These formulations often are multiple unit dosage forms which are presented in a single dose container containing the exact amount of formulation for one administration time point, which allows a high uniformity of dosing in contrast to existing formulations which are provided in multi-dose containers and dosed by patients themselves with a measuring cup.

As some individuals have a severe progression of the disease, even with a classical long term steroid and/or 5-ASA treatment, new formulations are also needed to provide better therapy. Other individuals do not favorably respond or become substantially irresponsive to steroid and/or 5-ASA treatment. For individuals having a 5-ASA-refractory disease, a removal of the colon can become necessary.

The present invention in particular relates to a pharmaceutical formulation comprising a particular phosphatidylcholine product (PC) for ameliorating at least one symptom of ulcerative colitis. This particular, the phosphatidylcholine product (PC) is combined with specific excipients for a delayed release formulation, often as a multiple unit dosage form, presented in a single dose container.

In one embodiment, the pharmaceutical formulation of the invention comprises:
a) at least one phosphatidylcholine product (PC) of formula (I), 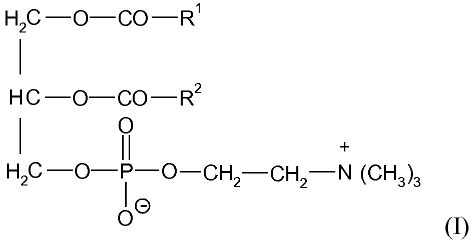 wherein:
   R¹ is the residue of a saturated or unsaturated fatty acid with 12 to 24 carbon atoms, and
   R² is the residue of a saturated or unsaturated fatty acid with 12 to 24 carbon atoms. R¹ and
   R² often are residues of saturated or unsaturated fatty acid with 14 to 22 carbon atoms.

The two fatty acid chains R¹ and R² in formula (I) are often not the same. The percentage of unsaturated fatty acid residues for R¹ in one embodiment of the invention is lower than for R², e. g. R¹ has less than 70% (by weight) unsaturated fatty acids whereas R² has more than 90% unsaturated fatty acids.

The phosphatidylcholine product (PC) in general contains the following amounts of fatty acids (in weight percent of the total amount of fatty acids in the PC):

| | |
|---|---|
| 55 - 72 | linoleic acid |
| 10 - 18 | palmitic acid |
| 07 - 15 | oleic acid |
| 02 - 08 | linolenic acid |
| 02 - 08 | stearic acid. |

The phosphatidylcholine product (PC) can in addition contain small amounts of other fatty acid residues with 12 to 24 carbon atoms (e. g. up to 1.5 % by weight).

The phosphatidylcholine product (PC) preferably denotes at least 94 % by weight of all lipid/phospholipid components of the formulation.

The phosphatidylcholine product (PC) according to the present invention can also be described as "substantially free of phosphatidylinositol (PI) and phosphatidylethanolamine (PE)", corresponding to less than 0.1% w/w of phosphatidylinositol (PI) and phosphatidylethanolamine (PE), respectively, of the entire lipid/phospholipid components of the formulation.

In one embodiment of the invention, the phosphatidylcholine product (PC) used contains the following amounts of fatty acids, or it essentially consists of these fatty acids (in weight percent of the total amount of fatty acids in the PC):

| | |
|---|---|
| 59 - 70 | linoleic acid |
| 12 - 17 | palmitic acid |
| 07 - 15 | oleic acid |
| 03 - 07 | linolenic acid |
| 02 - 05 | stearic acid. |

Often, the phosphatidylcholine product (PC) used contains 10 to 15 % of oleic acid-residues, preferably also from more than 10 to 12 % of oleic acid-residues.

Furthermore, the pharmaceutical formulation of the invention comprises:
b) at least one pharmaceutically acceptable excipient (which is not a phospholipid).

Often, the pharmaceutical formulation of the invention comprise several (two or more) different excipients.

The phosphatidylcholine product (PC) used can e.g. be prepared from a soy lecithin by using known process method steps. The different excipients can either be bought or can be prepared by the skilled person.

The PC-containing formulation often contains as phospholipid-component mainly (≥ 90 %) phosphatidylcholine (PC). Often the lipid/phospholipid components consist to ≥ 94 % by weight, often to ≥ 95 % by weight, of phosphatidylcholine. They normally contain only small amounts of other lipid/phospholipid components. The lipid components (within the PC-containing formulation) preferably contain other phospholipid components in an amount of less than 0,1 % (such as (PE) and (PI)). In addition to the higher enrichment degree of the active PC in the PC-containing product, the active pharmaceutical ingredient PC is much better characterized than known lecithin products, including monitoring of fatty acid composition in the diglyceride phosphatidylcholine.

The invention in particular relates to a PC-formulation with pH controlled release that shows gastric acid resistance and assures a high PC release into the intestine.

It can be demonstrated by liquid chromatography / mass spectrometry (LC/MS) analysis that the lipid components contained in the formulation consist to ≥ 94 % by weight of PC and contain ≤ 3 % by weight of nonpolar lipids. The total lipid components within the formulation preferably have a content of ≤ 2 % by weight of (free) triglycerides and ≤ 3 % by weight of lysophosphatidylcholine. The total lipid components within the formulation preferably also have ≤ 0.1 % by weight of phosphatidylethanolamine (PE) and ≤ 0.1 % by weight of phosphatidylinositol (PI).

The pharmaceutical formulation in one embodiment of the invention releases at least 50 % by weight, in particular at least 70 %, preferably at least 80 %, of the phosphatidylcholine product (PC) into the intestine. This can be measured according to standard in-vitro-models (see European Pharmacopoeia 7.6, Method 2.9.3 or comparable methods).

The excipients of the pharmaceutical formulation are in a preferred embodiment chosen in a way that at least 50 %, preferably at least 70 %, more preferably at least 80 % by weight of the phosphatidylcholine product (PC) is released in the intestine of the individual as assessed by standard in-vitro-models. Preferentially, the excipients of the pharmaceutical formulation are chosen so that a high percentage (at least 50 %, preferably at least 70 %,) of the phosphatidylcholine product (PC) reaches the colon.

The pharmaceutical formulations of the invention as described are preferably oral formulations, but other application routes are in principle also possible. The pharmaceutical formulations of the invention often are provided in a single dose container, such as a sachet (e.g. containing the adequate number of PC-pellets for one administration time point.).

The invention also relates to a pharmaceutical formulation comprising at least one phosphatidylcholine product (PC) of formula (I), wherein R¹ is the residue of a saturated or unsaturated fatty acid with 14 to 22, in particular 14 to 20 carbon atoms, and R² is the residue of a saturated or unsaturated fatty acid with 14 to 22, in particular 14 to 20 carbon atoms, and wherein the phosphatidylcholine product (PC) contains the following amounts of fatty acids (in weight percent of the total amount of fatty acids in the PC):

| | |
|---|---|
| 59 - 70 | linoleic acid |
| 12 - 17 | palmitic acid |
| 07 - 15 | oleic acid, (often >10-12) |
| 03 - 07 | linolenic acid |
| 02 - 05 | stearic acid. |

Typical amounts of the mentioned fatty acids (in weight percent of the total amount of fatty acids in the PC) are:

| | |
|---|---|
| 62 - 68 | linoleic acid |
| 12 - 15 | palmitic acid |
| 07 - 12 | oleic acid, (often >10-12) |
| 05 - 07 | linolenic acid |
| 02 - 04 | stearic acid. |

Often, more than 78 %, in particular more than 80 %, by weight of the fatty acid residues R¹ and R² of the PC are unsaturated fatty acid with 18 carbon atoms.

The phosphatidylcholine product (PC) is preferably obtained from specific natural sources. These sources can be e.g. plants, such as soy beans. These sources can also be e.g. animals, such as egg yolk. The phosphatidylcholine product (PC) however can also be obtained by chemical synthesis. Methods for the chemical preparation of phosphatidylcholine products with specific fatty acid residues are known to the chemist.

The PC can contain small amounts of other fatty acid residues, e. g. a total amount from 0.1 to 1.5 % (by weight), in particular from 0.1 to 1.0 %, preferably less than 1 % of other saturated or unsaturated fatty acids, in particular with 14 to 22 carbon atoms.

One manufacturing process for the specific PC-product can be described as following, where the starting material is soybean:

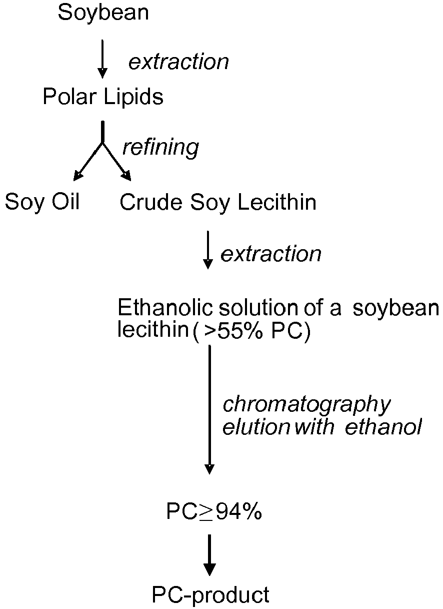

The invention also relates to a pharmaceutical formulation, comprising at least one phosphatidylcholine product (PC) of formula (I), wherein at least 75 %, in particular at least 80 % by weight, often more than 80 % by weight of the fatty acid residues R¹ and R² are unsaturated fatty acid with 18 carbon atoms.

The invention also relates to a pharmaceutical formulation, wherein the pharmaceutical formulation consists of or comprises enteric coated pellet(s), enteric coated tablet(s), enteric coated capsule(s) or an enteric coated granule(s), comprising the phosphatidylcholine product (PC) and at least one layer of at least one enteric polymer coating, which is resistant to gastric acids. Resistant to gastric acid means that at pH-value of 1, after 2 hours, less than 10 percent of PC is released from the coated PC-formulation.

The invention in particular relates to a pharmaceutical formulation as a multiple unit dosage form (comprising several enteric coated pellets) presented in a single dose container (such as a sachet) and containing the exact pellet amount for one dosing time point).

The invention also relates to a pharmaceutical formulation, wherein the weight ratio of enteric polymer coating and phosphatidylcholine product (PC) is from 10 : 1 to 1 : 10, often from 5 : 1 to 1 : 5. In some embodiments, this ratio is from 3 : 1 to 1 : 3, in particular from 2 : 1 to 1 : 2.

The pharmaceutical formulation often has a weight ratio of enteric polymer coating and phosphatidylcholine product (PC) from 3 : 1 to 1 : 3 and has the PC-product in a multiple unit dosage form. This weight ratio of enteric polymer coating and phosphatidylcholine product (PC) was found to prevent some side effects, such as flatulence.

For some embodiments, in addition to the enteric polymer coating, further excipients are used (such as hypromellose or amylose), in order to form a core together with the PC which protects the active ingredient.

The invention also relates to a pharmaceutical formulation, comprising an (or several) enteric coated pellet(s), comprising enteric coated pellet(s) or enteric coated capsule(s) or enteric coated granule(s) or enteric coated tablet(s), having an enteric polymer coating with a thickness of 10 to 800, in particular from 10 to 500 micrometers. The thickness of the coating of pellets is often from 10 to 300 micrometers, the thickness of the coating of capsules is often from 50 to 800 micrometers. The combination of specific pellet-coating thickness and PC-product consistence leads to increased stability.

The invention also relates to a pharmaceutical formulation comprising the phosphatidylcholine product (PC) protected by at least one enteric polymer coating, which is resistant to gastric acids (pH 1) for at least 120 minutes (release of PC lower than 10 %), but which allows at least 80 % of the phosphatidylcholine product (PC) to be released from the formulation at a pH of 5.5 or higher within 120 minutes.

The invention also relates to a pharmaceutical formulation, comprising at least one enteric polymer from the group of homo-polymers and co-polymers of acrylic acid, methacrylic acid, acrylic esters and methacrylic esters. Also mixtures of homo-polymers and co-polymers can be used. The preferred polymers for use as enteric excipients (such as Eudragit® L30-D55 or L100-55) are described later.

The invention also relates to a pharmaceutical formulation, comprising from 20 to 30 % by weight of a phosphatidylcholine product (PC) of formula (I) and 70 to 80 % by weight of excipients (total). The weight of the excipients is measured in the dry form. In some types of formulations, such as coated capsules, the amount of phosphatidylcholine product (PC) can be higher, such as e. g. up to 70 % by weight. Often 35 to 70 % by weight of PC are combined with 55 to 30 % by weight of excipients in these coated PC-capsules.

The invention also relates to a pharmaceutical formulation, having a long term stability, characterized by the amount of at least 95 % by weight of Assay PC (relative to nominal value) and less than 5 % by weight of Lyso-PC (relative to PC) after 36 months at refrigerated storage (5° C plus/minus 3° C).

One further aspect of the invention is a process for the preparation of a pharmaceutical formulation as described above, comprising the step of preparing pellet(s) or tablet(s) or capsule(s) or granule(s), containing at least one phosphatidylcholine (PC) of formula (I), 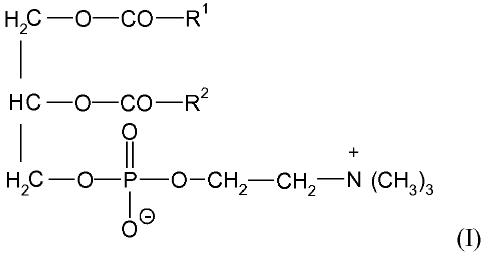 wherein R¹ and R² are defined as above, wherein the phosphatidylcholine product (PC) contains the following amounts of fatty acids (in weight percent of the total amount of fatty acids in the PC):

| | |
|---|---|
| 55 - 72 | linoleic acid |
| 10 - 18 | palmitic acid |
| 07 - 15 | oleic acid |
| 02 - 08 | linolenic acid |
| 02 - 08 | stearic acid. |

and then providing the pellet(s) or capsule(s) or granule(s) or tablet(s) with at least one layer of an enteric polymer coating. The preferred PC-products (with the more precisely defined fatty acid residues) are to be prepared by this method as described as above.

One further aspect of the invention relates to a formulation as described above for the treatment or prevention of a condition or disease from the following:
ulcerative colitis, Crohn's disease, diversion colitis, infectious enteritis, infectious colitis, inflammation due to irradiation and inflammation due to chemotherapeutics or chemicals, in particular for the treatment of ulcerative colitis in 5-ASA-refractory patients.

The formulations are in particular used for the treatment (including maintenance of status) or prevention of ulcerative colitis, Crohn's disease, or ulcerative colitis in 5-ASA-refractory or steroid-refractory patients.

They can also be used for the treatment or prevention of ulcerative colitis in 5-ASA-refractory patients, where a dose of 0.5 to 8 g, in particular 2 to 6 g, of a phosphatidylcholine product (PC) of formula (I) per day is applied. This dosage is often applied in a single dose container (e.g. sachet), containing the adequate amount of multiple unit dosage form (e.g. PC-pellets) for one dosing time point. For the treatment or prevention of ulcerative colitis in 5-ASA-refractory patients, the daily dose of phosphatidylcholine product (PC) of formula (I) can be applied (in portions, often in equal portions) twice or three times or four times per day, often three times or four times per day.

In one embodiment, the formulation (e.g. enteric formulation) comprising the phosphatidylcholine product (PC) of formula (I) is applied to an individual having ulcerative colitis, whereby a therapeutically effective amount of phosphatidylcholine (PC) is used to ameliorate at least one symptom of the ulcerative colitis.

In another embodiment, the present invention relates to a formulation for ameliorating at least one symptom of ulcerative colitis in an individual having 5-ASA-refractory or steroid-refractory ulcerative colitis.

By using standard oral formulations, the phosphatidylcholine product, or at least a significant part of the PC comes in contact with the stomach surface and will be decomposed. In order to minimise the interaction of the PC with the animal or human stomach, the release of the specific PC can be directed in the small or large intestine, in particular the colon. For the targeted release according to this invention, several formulation technologies can be applied. One particular formulation technology for delayed release is the preparation of enteric formulations, such as coated tablets, coated capsules, coated pellets or coated granules. In particular, the enteric coated PC-pellets will reduce the degradation of PC and systemic uptake in the stomach and upper gastrointestinal tract.

Enteric formulation in this context means in particular, that a composition (e.g. granules or pellets or capsules) comprising the PC is combined with, e. g. coated with a material that permits a transit of the PC through the acidic stomach with only limited or without any release of PC in the stomach. The PC is transported into the intestine or into a special part of the intestine before the PC is released at a pH of 5.5 or higher. The term "enteric" means in particular that the release of the PC is triggered either in the small intestine or in the large intestine or in both compartments. The selection of the optimal release site depends on the intended local concentrations, the intended time/concentration profile at the target site (e. g. colon) of action for the PC.

The term "excipient" shall mean a pharmacologically inactive substance which is used as a carrier for the active substance and/or the design of formulations of drug products. The term "excipient" shall include a pharmaceutically acceptable, pharmacologically inactive ingredient such as a binder, a filler, a coating-forming compound, a plasticizers for coatings and a compound which masks odors. Some examples of optional excipients are pigments, disintegrants, antioxidants, flavors, sweeteners, colourants, opacifiers, anti-adhesives, preservatives, glidants, lubricants, sorbents and isolating-layer forming agents. Suitable substances are known in the art. The term "excipient" applied to pharmaceutical formulations of the invention also refers to a diluent or vehicle with which an active substance is administered. Such pharmaceutical excipients can be from animal, vegetable or synthetic origin, see also A. R. Gennaro (20th Edition in "Remington: The Science and Practice of Pharmacy", 2001).

The term "capsule" shall mean a pharmaceutically acceptable case enclosing a dose of one active substance or a combination of active substances and one excipient or a combination of excipients, which is covered by a polymer shell, which e. g. basically consists of gelatine, starch or cellulose or chemical derivatives and combinations of these polymers. Capsules can be soft or hard capsules. Their content can be solid, semi solid or liquid.

The term "granule" shall mean aggregates of particles, e.g. powder particles, to form a multi-particle entity. In pharmaceutical terms, a granule encompasses small particles gathered into a larger, permanent aggregate in which the original particles may still be identified. Granules may be obtained in a granulation process in which powder particles adhere to each other by different physical mechanisms. Processes such as thermoplastic granulation, aqueous or organic solvent based pot granulation, granulation in a tumbling mixer, granulation in a fluidized bed granulator, granulation by spray drying or dry granulation by compaction are known in the field of pharmaceutical compositions.

The term "immediate release" shall mean a release rate in which at least 80 % of the active substance is released after 30 minutes after oral application of the formulation. Experimental conditions for measuring the release are the conditions as defined in U.S. Pharmacopoeia, e.g. USP 35, Method 711 (2012), see also corresponding European Pharmacopoeia, e.g. EP 7.6.

Contrary to this definition, the term "delayed release formulation" encompasses a dosage form which releases an incorporated active substance in a timely delayed and/or controlled way and/or rapidly or slowly and in a defined part of the gastro intestinal tract over a period of time as defined in detail. This term encompasses a pharmaceutical formulation comprising a therapeutically effective amount of the active substance (or a pharmaceutically acceptable salt, solvate, polymorphic form or isomer thereof) and at least one release delaying excipient. The term encompasses enteric coating formulations.

The term "multiple unit dosage form" encompasses a dosage form which consists of at least two units which contain the effective amount of the PC. The term "single unit dosage form" encompasses a formulation which consists of only one unit which contains the effective amount of the phosphatidylcholine.

However, often multiple unit dosage forms are presented in a single dose container, such as a sachet containing the amount of PC pellets for one administration time point.

In one embodiment of the invention, the formulation is in the form of a (single) sachet comprising 0.5 to 8.0 g of PC in the form of pellets, in particular about 3.2 g or 6.4 g of pellets, in particular for the daily treatment of ulcerative colitis.

The term "pellet" shall mean a spherical particle typically created by special granulation technologies. A pellet may be produced by layering active material on a starter particle or by extrusion and spheronisation or by pelletizing in a fluidized bed or by thermal melting, forming, cooling processes. A pellet may also be produced by granulating the excipients with an aqueous dispersion of the active material, followed by extrusion, drying, coating etc. Such processes for producing pellets are known in the field of pharmaceutical formulation development.

The term "pharmaceutically acceptable" in connection with a substance shall mean an ingredient or a substance which does not affect the safety of a human being and/or is well-tolerated by a human being after administration. The term "polymorphic form" encompasses an active substance, a pharmaceutically acceptable salt, solvate or isomer thereof forming different crystal structures or lattices.

The term "tablet" shall mean any solid pharmaceutical composition comprising the active substance. The term encompasses both compressed formulations and non-compressed formulations. Non compressed formulation can be manufactured e.g. by thermal or melting processes. The tablet may have any shape, which is common in the field of tablets, such as a round shape, a rectangular shape or an oval shape, or a convex shape, or the shape of a disk, or the shape of a bead. The shape may also be irregular. The term also comprises the term "mini-tablet" and "micro-tablet". Such term is known in the field of pharmaceutical compositions. A tablet may be made from granules and/or pellets. The processing of granules and or pellets into tablets is known to a person skilled in the art.

The invention also relates to pharmaceutical formulations comprising at least one PC-product of formula (I) as described above and at least one excipient for the delayed release for the phosphatidylcholine. These PC-formulations considerably reduce the undesired systemic uptake of the PC, reduce the degradation of the PC and enable a high dosage of the phosphatidylcholine, administered orally, to arrive in the colon. These pharmaceutical formulations also have a high degree of stability (over time during storage).

The phosphatidylcholine (PC) of the above mentioned formula (I) can be administered in a once-daily delayed release formulation, preferably in an oral once-daily delayed release formulation, but also twice daily, four times daily and other formulations are possible.

In one embodiment, the delayed release of the phosphatidylcholine (PC) is pH-dependent. The pH-dependent delayed release formulations can comprise one or more excipients, preferably in form of a layer surrounding the PC or the core containing the PC, which are resistant to gastric acids (e.g. pH 1.0) for several hours, such as 2 hours or more.

The PC-containing pellets according to this invention can comprise the PC described above and binders (such as cellulose) and disintegrants (such as silica) and other excipients known for the preparation of such formulations.

In one embodiment, the pH-dependant coating also contains an amylose based component, which can be broken down specifically by the microorganisms in the colonic region. The power of these amylose based components in coatings has been demonstrated in a scintigraphic study by Ibekwe (Alimentary Pharmacology & Therapeutics 28, 2008). Capsules with a PC-core or PC-pellets with the combination of Eudragit® (e.g. L30-D55) and amylose coatings are disintegrated in the colonic area as desired. In one embodiment, the pH-dependent delayed release formulation of the PC contains at least one release-controlling excipient, which is a polymer selected from the group of:
Homo-polymers and co-polymers of acrylic acid,
methacrylic acid, acrylic esters and methacrylic esters.

In one embodiment, small amounts (up to 10 %) of other monomers can also be used in the copolymer compositions. Also mixtures of homo-polymers and co-polymers can be used for delayed release layers. The preferred polymers for use as enteric excipients are commercial products of the type such as Eudragit® L-30-D55.

In one embodiment, the formulation of the PC comprises a second excipient, e. g. hydroxypropylmethyl-cellulose (hypromellose) or a polyvinyl alcohol or polyvinyl acetate or a polyvinyl alcohol grafted with polyethylene glycol.

Further suitable components and polymers for the enteric formulations are release-controlling and modifying lipids and waxes, for example, beeswax, natural or synthetic mono-, di- and triglycerides of medium and long chain fatty acids.

In one further embodiment, the formulation of the PC comprises shellac as an excipient.

Some release-controlling excipients based on polymethacrylate and/or poly-meth-acrylate copolymers are e.g. commercially available under the trademark Eudragit® (of Evonik Industries, Germany). The following polymers can be used to prepare e. g. enteric pellets or granules containing the PC: Eudragit® L and/or Eudragit® S. Typical grades are e.g. Eudragit® L 30, Eudragit® L30-D55. Other grades are Eudragit® S100, Eudragit® FS, Eudragit® RS 30 D, Eudragit® RL 30 D, Eudragit® NE 40 D, Eudragit® RS PO and Eudragit® NE 30 D, Eudragit® SS, Eudragit® L100-55 or a combination of two or more thereof.

Eudragit® L30-D55 contains an anionic copolymer based on methacrylic acid and ethyl-acrylate. Eudragit® L30-D55 is an aqueous dispersion, containing 30 % by weight of the copolymer. This coating material is preferred.

Eudragit® S 100 contains an anionic copolymer based on methacrylic acid and methylmethacrylate.

Eudragit® RL 30 contains from 10.18 to 13.73 % ammonia methacrylate moieties based on dry substance determined according to Ph. Eur. 2.2.20.

The relative amount of enteric coating needed in the PC-formulation to achieve the desired release characteristics depends, inter alia, on the selected polymer type and grade, the presence or absence of other excipients having impact on release of active substance, and on the desired drug load.

The weight ratio of this enteric polymer (calculated to the dry weight) to the active substance (PC) is typically selected in the range from 100 : 1 to 1 : 100, or from 50 : 1 to 1 : 100, or from 10 : 1 to 1 : 100. In one particular embodiment, the ratio of polymer excipient to active substance is from 5 : 1 to about 1 : 5, often from 3 : 1 to about 1 : 3 (weight/weight).

For example, the gastric acid (pH 1) resistant substances can comprise coating and carrier materials, such as the polymers/copolymers sold as Eudragit®. The commercial products Eudragit® L and/or Eudragit® S and/or Eudragit® FS, and especially the polymer Eudragit® L30 can be used as excipients. Exemplary preferred pH-dependent delayed release formulations comprise 0.5 to 10.0 g of pellets per single sachet, containing the phosphatidylcholine (PC), coated with a polymer such as Eudragit® L30-D55 (or Eudragit® S100, Eudragit® FS or a mixture of these polymers).

In embodiments of the above-described formulation, the therapeutically effective amount of phosphatidylcholine product (PC) that is administered to the individual ranges from 0.5 g to 8 g per day. A preferred range includes from 2 g to 5 g per day. In another preferred embodiment, the therapeutically effective amount of phosphatidylcholine that is administered to the individual is about 3 to 4 g per day.

According to one embodiment, the formulation comprising the phosphatidylcholine is administered shortly before meal time, in particular at least 60 minutes (such as 60 to 90 min) before meal. The frequency of administration of the formulation of the phosphatidylcholine can be from 1 to 8 times per day with a preferred administration frequency being 2 to 4 times per day. Administration of the formulation may be continued for at least 1 week, at least 2 weeks, at least 2 months, at least 3 months or as long as needed throughout the life of the individual. In some embodiments of the present invention, administration of the PC-formulation above-described can result in a quality of life index score improvement of at least 25 %, a histology index reduction of at least 25 %, an endoscopic activity index improvement of at least 25 %, a clinical activity index improvement of at least 25 %, or in some cases, cessation of clinically active ulcerative colitis.

Other embodiments of the present invention relate to a formulation of the specific phosphatidylcholine (PC) for ameliorating at least one symptom of ulcerative colitis in an individual identified as having 5-ASA-refractory or steroid-refractory ulcerative colitis.

In certain embodiments of the formulation described above, the phosphatidylcholine product (PC) may be formulated as a delayed release formulation for oral administration wherein the delayed release is not pH-dependent, but time dependent, e.g. releasing the PC only after 3 to 4 hours after oral administration. G. Fiorino (Current Medicinal Chemistry, 2010, 17, 1851-1857) describes such types of coatings for Mesalazine. Also L.F.A. Asghar describes multi-particle formulations for colon directed drugs (J. Pharm Pharmaceut. Sci, 9 (3), 327-338; 2006).

The oral formulations with the delayed release can be prepared by coating the phosphatidylcholine containing pellets (or cores) with one or several layers of polymers which are gastric acid resistant and release the phosphatidylcholine in pH-dependent fashion into the intestine, such as lower ileum or colon. In a preferred embodiment, one or two layers comprising or consisting of pH-dependent delayed release polymers, such as Eudragit®, are applied on the PC-containing core. In particularly, Eudragit® L 30-55 and/or Eudragit® S are used to coat the phosphatidylcholine-pellet (or core). In some embodiments, the outer coating comprises Eudragit® L30-55 and/or Eudragit® L30D.

The oral pH-dependent delayed release formulations can be manufactured e. g. as pellets, granules, capsules or tablets. Some colon-targeting drug formulations were described by M.K. Chourasia (J. Pharm. Pharmaceut. Sci 6(1); 33-66; 2003).The formulations according to this invention can further contain the usual pharmaceutical excipients including binders, diluents, carrier substances, flow agents, pigments, disintegrants, antioxidants, flavors, sweeteners, colourants, opacifiers, anti-adhesives, preservatives, glidants, lubricants, sorbents and isolating-layer forming agents.

In a preferred embodiment of the present invention, sachets (or other containers) holding pH-dependent delayed release pellets (or granules) comprising the phosphatidylcholine (PC) are provided, e.g. containing 2 to 10 g of PC-pellets.

In another embodiment, the phosphatidylcholine of formula (I) is packed in a high volume in gelatin capsules. The gelatin capsules are then coated with at least one layer of Eudragit® or another acrylate or methacrylate polymer for pH-dependent delayed release. These capsules often encompass higher amounts of PC in relation to the excipients (e.g. 65 % to 35 % by weight) than e. g. the pellets described.

The efficacy of the formulations described above can be assessed by monitoring treated individuals using indices for the assessment of the severity of ulcerative colitis that are known in the art. These indices include, but are not limited to, the clinical activity index (CAI), the endoscopic activity index (EAI), the histology index (HI), the Simple Clinical Colitis Activity Index (SCCAI), Mayo Score and the life quality index (LQI or IBDQ-D). One skilled in the art knows such indices as common diagnostics used to evaluate the progression and/or efficacy of treatment of ulcerative colitis.

The Simple Clinical Colitis Activity Index (SCCAI) records the clinical activity by considering the number of bowel movements during day and night, urgency of defecation, presence of visible blood in the stool, general well being, abdominal pain, and extra-intestinal manifestations. This score mainly considers the clinical activity of ulcerative colitis which was shown to correlate with the endoscopic activity. It can be performed by the investigator interviewing the patient or the patient completing a questionnaire comprising questions about his disease.

The Mayo Score, also known as Mayo Clinic Score or Disease Activity Index, is a known instrument for the evaluation of ulcerative colitis. It consists of four items: stool frequency, rectal bleeding, mucosal appearance, and a physician's global assessment (PGA). The score ranges from 0 to 12 points. Over the years of use, this known score has been modified to adapt it to modem medical knowledge. This "modified Mayo Score" has already been used in other clinical studies and was demonstrated to be a reliable measure for changes in ulcerative colitis. The Quality of life (QOL) in patients suffering from ulcerative colitis can be measured by the Inflammatory Bowel Disease Questionnaire (IBDQ).

The performance of the formulations described for ulcerative colitis treatment can also be assessed by biochemical methods. Two specific biomarkers, Calprotectin and S100A12, belonging to the calcium-binding S100 protein family, are tissue specifically released by activated or damaged cells under condition of cell stress and therefore represent reliable, non-invasive markers of inflammatory activity. Calprotectin, a complex of the 2 phagocyte specific proteins S100A8 and S100A9, can be used in clinical trials to evaluate inflammatory activity and treatment response. Calprotectin can be found to correlate significantly with clinical and endoscopic activity in inflammatory bowel diseases. It is also a reliable surrogate marker for mucosal healing and a predictor of relapse in clinically quiescent ulcerative colitis. The second biomarker, S100A12 may have an even better diagnostic accuracy and a higher correlation to mucosal inflammation than Calprotectin.

In some embodiments of the formulations described, the administration of phosphatidylcholine product (PC) to an individual having 5-ASA-refractory ulcerative colitis results in a endoscopic activity index (EAI) improvement of at least 10 %, at least 20 %, at least 30 %, at least 40 %, at least 50 %, at least 60 %, at least 70 %, at least 80 %, at least 90 % or 100 %.

In some embodiments of the formulations described herein, administration of a phosphatidylcholine product of formula (I) to an individual having 5-ASA-refractory ulcerative colitis results in a histology index reduction of at least 10 %, at least 20 %, at least 30 %, at least 40 %, at least 50 %, at least 60 %, at least 70 %, at least 80 %, at least 90 % or 100 %.

In some embodiments of the formulations described herein, administration of the phosphatidylcholine product to an individual having 5-ASA-refractory ulcerative colitis results in a clinical activity index improvement of at least of at least 10 %, at least 20 %, at least 30 %, at least 40 %, at least 50 %, at least 60 %, at least 70 %, at least 80 %, at least 90 % or 100 %.

In some embodiments of the formulations described herein, administration of the phosphatidylcholine to an individual having 5-ASA-refractory ulcerative colitis results in a quality of life index improvement of at least 5 %, at least 10 %, at least 15 %, at least 20 %, at least 25 %, at least 30 %, at least 35 %, at least 40 %, at least 45 %, at least 50 %, at least 55 %, at least 60 %, at least 65 %, at least 70 %, at least 75 %, at least 80 %, at least 85 %, at least 90 %, at least 95 %, or 100 %.

The phosphatidylcholine products (PC) of formula (I), e. g. those containing (or consisting essentially of) the following amounts of fatty acids (in weight percent of the total amount of fatty acids in the PC):
62-66 linoleic acid
12-15 palmitic acid
>10-12 oleic acid
05-07 linolenic acid
02-04 stearic acid,
can easily be prepared in high purity in large amounts (e. g. 100 kilogram).

The invention is further illustrated in the patent claims and by the following Examples.

### Example 1a (Formulation of PC as pellets)

A pharmaceutical formulation is prepared in the form of pellets (diameter of 0.5 to 1.8 mm), containing 27 % by weight of a specific phosphatidylcholine product (PC) of formula (I), containing the following amounts of fatty acids (in weight percent of the total amount of fatty acids in the PC):

| | |
|---|---|
| 64 | linoleic acid |
| 14 | palmitic acid |
| 11 | oleic acid |
| 06 | linolenic acid |
| 04 | stearic acid |
| 01 | other C-14 to C-22 fatty acids |

This phosphatidylcholine product (PC) has a high degree of purity and does contain less than 0.1 % of (PE) and (PI). This PC product can be obtained by chemical preparation or by the process described before from soybean lecithin and was formulated with the following excipients in following proportions (weight percent) where the firstly named components were used to prepare the PC-pellet (core) and the secondly named excipients were used for the delayed release layer:
A) For the PC-Core:

| | |
|---|---|
| 26.96 | PC (including a small amount of α-Tocopherol) |
| 0.03 | Ascorbyl palmitate (Antioxidant) and/or α-Tocopherol |
| 34.09 | Microcrystalline cellulose (Avicel® PH102; Binder) |
| 4.88 | Colloidal hydrated silica (Syloid® 244FP; Binder) |
| 1,39 | Colloidal anhydrous silica (Carb-O-Sil M5P; Disintegrant) |
| 2.08 | Croscarmellose sodium (Ac-di-Sol; Disintegrant). |

B) For the Coating:

| | |
|---|---|
| 1.21 | Hydroxypropyl-methyl-cellulose (Hypromellose, Pharmacoat 606) |
| 0.05 | Polyethylenglycol (Macrogol 6000; Plasticiser) |
| 2.68 | Talc (Anti-adherent) |
| 24.16 | Eudragit® L 30 D-55 (Film-former) |
| 2.47 | Triethyl citrate (Plasticiser). |

The phosphatidylcholine product (PC) of the core contains at least 97 % of acetone-insoluble matter. The core of the pellets contains the phosphatidylcholine described above in highly purified form (with less than 0.1 % by weight of phosphatidylethanolamine and less than 0.1 % by weight of phosphatidylinositol). For the preparation of the orally to be applied phosphatidylcholine formulation, the pellets are covered with a layer of acrylate-copolymer, Eudragit® L30-55, which ensures a delayed release at pH > 5.5 in the small intestine. The pellets having a size of 0.5 to 1.4 mm (diameter) were pre-isolated with a hypromellose containing layer and subsequently coated with an anionic copolymer based on methacrylic acid and ethyl-acrylate polymer (Eudragit® L30-55). The final coated pellets have a diameter of up to 1.8 mm.

### Example lb (Batch preparation of verum pellets (C103) and placebo pellets (C104))

The formulation can for example be prepared in a technical scope as following:

| | Verum Pellets (CLT-103) | PlaceboPellets (CLT-104) |
|---|---|---|
| | [kg/batch] | [kg/batch] |
| Components | Scale: | Scale: |
| | 120 kg | 250 kg |

| | Verum Pellets (CLT-103) | PlaceboPellets (CLT-104) |
|---|---|---|
| | [kg/batch] | [kg/batch] |
| Components | Scale: | Scale: |
| | 120 kg | 250 kg |
| Composition of the pellets | | |
| Phosphatidylcholine product | 32.350 | --- |
| Ascorbyl palmitate | 0.034 | --- |
| Microcrystalline cellulose | 40.906 | 34.717 |
| Colloidal hydrated silica | 5.858 | 17.357 |
| Colloidal silicon dioxide | 1.667 | 3.473 |
| Croscarmellose sodium | 2.499 | 5.207 |
| Crospovidone | | 86.777 |
| Potato starch | | 26.037 |
| Riboflavin | | 7.500 |
| Purified water¹ | 37.491 | 266.477 |

Batch formula of the film coating for pre-isolation with isolation lacquer (with stated amounts include 10% production overage for all excipients needed for the film-coatings).

| | | |
|---|---|---|
| Purified water¹ | 37.328 | 77.767 |
| Hypromellose | 1.595 | 3.323 |
| Polyethylen glycol 6000 | 0.072 | 0.150 |

| | Verum Pellets (CLT-103) | PlaceboPellets (CLT-104) |
|---|---|---|
| | [kg/batch] | [kg/batch] |
| Components | Scale: | Scale: |
| | 120 kg | 250 kg |
| Talc | 0.715 | 1.490 |

Batch formula of the film coating with gastric juice resistance lacquer (see above)

| | | |
|---|---|---|
| Eudragit L 30 D-55 | 106.322 | 221.503 |
| Triethyl citrate | 3.254 | 6.780 |
| Purified water¹ | 77.464 | 161.383 |
| Talc | 2.821 | 5.877 |

| | | |
|---|---|---|
| 1) The water is removed during manufacture, not present in the final formulation. | | |

### Example 2 (Release of PC and Stability)

The pellets containing the specific phosphatidylcholine product (PC) according to examples 1 were tested as to the stability under various conditions. The release of PC under acidic conditions (pH 1.0; 0.1N HCl) was less than 10 % by weight after 120 minutes. The release over all three test media after further 60 minutes at ph 6.0 and after further 60 minutes at pH 7.2 was more than 75 % by weight.

The enteric coated pellets of example 1 were packaged into sachets and were tested in a classical stability study. The acceptance criteria were defined as 90 - 110 % of Assay PC (relative to nominal value) and a maximum of 5 % Lyso PC (relative to PC).

A first batch of the given formulation was tested after 36 months at refrigerated storage (5 °C, plus/minus 3 °C) and the pellets were found to be stable. The following test results were found: (Assay PC (relative to nominal value) = 96 % and Lyso-PC (relative to PC) = 3.35 %.

For another batch, throughout 12 months of storage at room temperature (25 °C plus/minus 2 °C) the pellets were found to be stable. After 12 months, the following test results were found for the key parameters PC content and Lyso-PC as its main degradation product: Assay PC (relative to nominal value) = 97 % and Lyso-PC (relative to PC) = 4.97 %

The known retarded release phosphatidylcholine formulations (Stremmel et al.) were found to be considerably less stable. After 4 months at refrigerated storage (5 °C, plus/minus 3 °C) the granule showed a content of Lyso-PC (relative to PC) = 13 %. After 3 and 6 months of storage at room temperature (25 °C plus/minus 2 °C) test results in following ranges were found: Assay PC (relative to the initial value) between 97.8 to 85.9 % and Lyso-PC (relative to PC) between 9.6 to 14.0 %.

### Example 3 (Pharmacological Testing)

The pellets containing the specific phosphatidylcholine product (PC) of Example 1 were tested in patients with ulcerative colitis to show the efficacy and safety of modified-release PC in mesalazine-refractory ulcerative colitis. In a multi-centre phase IIb study, a placebocontrolled, parallel-group, 4-arm design was used, including 156 patients. In four independent randomized arms placebo and daily doses of 0.8 g, 1.6 g, and 3.2 g of PC were applied.

The pellets contained in one sachet had to be taken four times a day orally with water, or, after mixing with water, juice, milk, or yoghurt immediately prior to administration. The formulation of example 1 was used for all dose strengths as delayed-release coated pellets. The amount of pellets of one dosage unit (sachet) was about 3.2 g of pellets.

For the highest PC dose strength, sachets contain verum pellets only, lower doses are obtained by mixing verum pellets and placebo pellets (containing as filling excipient crospovidone and potato starch instead of the PC-product) prior to filling the sachet, whereas PC-placebo sachets contain solely placebo pellets. The fill weight was identical for all PC formulations.

All patients treated with the specific phosphatidylcholine product (PC) or placebo had provided a written informed consent for this study before starting the trial. The study was initiated with a screening visit for general eligibility and randomization took place after a one week period which suited for determining the baseline activity of ulcerative colitis (according to SCCAI) and compliance with all other inclusion/exclusion criteria. Patients which fulfilled all criteria started treatment on the same day and took the PC medication for all together 12 weeks in a double blinded fashion.

The 12-week treatment period included 2 interim study visits and an end-of-treatment visit. Patients who completed the full 12 weeks treatment and went into partial or complete remission at end-of-treatment visit were then followed up for another 8 weeks or until relapse occurred. A Safety Telephone Visit was performed 4 weeks after the end of treatment for all patients who took the PC study medication at least once.

The clinical phase IIB study was conducted to demonstrate efficacy and safety of PC as described in examples 1. During the study certain colitis-related co-medication was allowed if continued at baseline value, defined as a stable dose at least 4 weeks prior to entering the study. Thus, a portion of the patients received mesalazine or sulfasalazine (5-ASA) during the study. In addition, patients were also allowed co-medication with steroids and immunosuppressants such as azathioprine. The clinical data demonstrate that daily oral administration of PC is well-tolerated and clinically active in patients with ulcerative colitis. The specific formulation was found more efficacious in ulcerative colitis than former phosphatidylcholine formulations used in previous studies.

One important result of the study was an overall large treatment effect in all study groups. Surprisingly the treatment effect in the 3.2 g/day PC dose group according to the prespecified biometrical analysis procedure led to a significant higher treatment effect over placebo.

The disease activity in the 3.2 g/day PC group decreased by 4.3 points, compared to 3.0 points with the placebo group, translating into a statistically significant treatment effect over placebo (with a p-value of 0.0298). Remission rates were higher in the PC treatment groups compared to placebo. At the dose of 3.2 g PC /day the number of patients going into complete or partial remission (which means that the clinical status of the patients was normal or close to normal) was 30 % as compared to the placebo group where only 15 % of patients went into remission.

Further analysis revealed that the co-medication for ulcerative colitis which was allowed during the study seemed to have an impact on the clinical outcome.

Especially the patient group without co-treatment with 5 ASAs (e.g. mesalazine) benefited substantially in clinical endpoints for response (clinical scores, endoscopy, histology). These holds true also for the remission rates which at the highest dose of 3.2 g/day PC reached 60% in comparison to a 10% response rate for patients receiving placebo. It is noteworthy that a standard marker for inflammatory activity called C-reactive protein (CRP) was high and in the pathological range in all patients at baseline. At the end of treatment all patients receiving PC treatment had a remarkable improvement in this laboratory parameter when compared to patients receiving placebo indicating a decrease in inflammatory activity caused by PC treatment.

Besides these excellent results in improving the clinical status of the ulcerative colitis patients receiving PC treatment, it is very impressive that the side effect profile of this formulation was very benign. There were no safety findings (vital signs, physical examination, safety laboratory) which were in any way dose related. The clinical data demonstrate that daily oral administration of PC as described in example 1 is well-tolerated and clinically active in patients with ulcerative colitis. The specific formulation was found very efficacious in the treatment of ulcerative colitis. The formulation as described showed less side effects and a better stability profile than former phosphatidylcholine formulations used in previous studies.

### Example 4 (Formulation of PC as capsules)

As a second controlled release formulation containing the specific PC of example 1 as active ingredient, capsules are prepared by using standard commercially available hard gelatine capsules (standard sizes 0 to 4). These are filled with either a mixture of the phosphatidylcholine product of example 1 and an oily excipient (Miglyol 812 at a weight ratio of 65 % PC / 35 % excipient) or with a mixture of the phosphatidylcholine product of example 1 (65 % by weight) and Tween 80 (17.5 % by weight) and Myglyol (17.5 % by weight). To provide a delayed release formulation for ulcerative colitis treatment, these gelatin capsules are coated with a layer of an anionic polymer based on methacrylic acid and ethyl-acrylate (such as the products Eudragit® described above).

### Example 5 (Technical Manufacturing Process)

| | | |
|---|---|---|
| Components of CLT-103 | Operations | Components of CLT-104 |
| Verum Bulk Pellets | | Placebo Bulk Pellets |

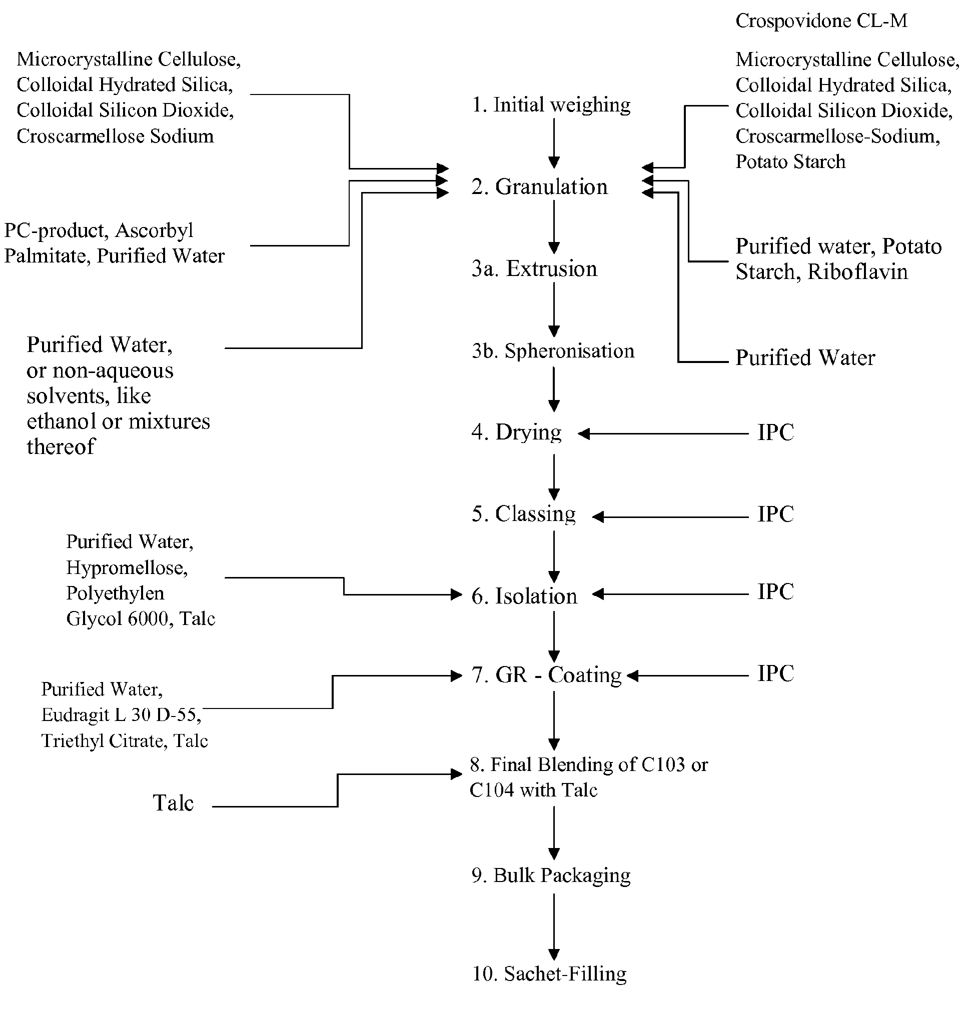

IPC means in-process-control.

Starting with Step 3a, the manufacturing processes of verum bulk pellets (C103), formulation according to the invention, and placebo bulk pellets (C104) are identical.

The following manufacturing equipment was applied:

| | |
|---|---|
| Manufacturing Steps | Equipment |
| Step 1 Initial weighing | Balance |
| Step 2 Granulation | Blade agitator Stainless steel container High shear mixer Diosna |
| Step 3 Extrusion / spheronisation | Extruder (NICA E220) Spheroniser (NICA S700) |
| Step 4 Drying | GPCG or Aeromatic Fielder |
| Step 5 Classing | Screen 1.40 und 0.50 mm |
| Step 6 Film coating with isolation film | Blade-agitator Stainless steel container Aeromatic Fielder |
| Step 7 Film coating with gastic-acid resistant film | Blade-agitator Stainless steel container Aeromatic Fielder |
| Step 8 Blending of verum or placebo pellets with talc | Hand screen 1.00 mm Freefall blender Container |
| Step 10 Sachet filling | Filling machine (LA 300). |

The manufacturing operations are carried out in conformance with the GMP rules. The manufacturing method consists of the following conventional steps: manufacturing of the granulation solvent, granulation, extrusion and spheronisation, drying, classing and film coating. Subsequently, blending of the verum bulk pellets (C103) or the placebo bulk pellets (C104) with talc is performed after addition of 2.5 % talc to obtain the final pellet mixtures suitable for sachet filling.

The PC-product is dispersed in a stainless steel container in warm water (40 °C-50 °C) and stirred for a defined time. Afterwards ascorbyl palmitate is added and stirring is continued. The resulting dispersion is used as granulation solution.

A mixture of microcrystalline cellulose, colloidal hydrated silica, colloidal silicon dioxide and crosscarmellose-sodium is placed into a Diosna and mixed. Afterwards the granulation solution is added and mixing will be continued. The standing time for the solution at this manufacturing step is limited to twelve hours. After granulation the granule is transferred into a stainless steel container.

The granule is transferred into an extruder, where in a first step extrusion and subsequently spheronisation takes place. The resulting pellets are dried in a fluid bed drier to reduce the residual humidity. The dried pellets are sieved in order to remove pellets bigger than 1.40 mm and pellets smaller than 0.50 mm diameter. Yield of the resulting fraction with pellet size between 0.50 and 1.40 mm is determined. After sieving, the pellets are stored between 2-8 °C until further processing. The size distribution of the pellets is determined via sieve analysis, results are reported.

For preparation of the film coating solution for the pellets, hypromellose and polyethylene glycol 6000 are put into purified water and stirred. Subsequently, talc is added and stirring is continued. The pellets are coated with the isolation lacquer by fluidized-bed film coating.

Eudragit® L30 D-55 is put via a sieve into a blade agitator container. While stirring constantly, triethyl citrate is added, subsequently purified water is added and stirring continued properly. Finally, talc is added, and stirring of the resulting coating solution is further continued. The pre-isolated pellets are then coated with the gastric juice resistance lacquer by fluidized-bed film coating.

To the verum or placebo pellets, 2.5 % talc referring to the bulk pellet weight is added and blending is performed, in order to overcome electrostatic charging of the bulk pellets.

Subsequent to sample drawing for release testing of the final mixtures, the bulk pellet mixtures are packaged into polyethylene bags, desiccant bag, light shield bag and 13 L curtecs. The quantity per container is determined. The pellet bulk mixtures are stored between 2 °C and 8 °C.

The bulk pellet mixtures are preconditioned at room temperature for 1 hr before start of sachet filling. Subsequently, the mixtures are filled with nitrogen flushing into sachets with identical target filling weights for the formulation according to the invention and Placebo sachets, corresponding to a theoretical amount of 800 mg PC per sachet for the verum formulation.

The target filling weight for the sachets is hereby calculated basing on the theoretical PC content of the pellet mixture, taking into account the PC content and the water content of the API batch PC-product used for manufacture.

As proven by these experiments, this invention relates to formulations for the treatment of ulcerative colitis by using of particular phosphatidylcholine products and also by using new formulations with improved excipients. It was unexpected that by having higher purity of PC of more than 94 % and e.g. a high ratio (such as 3 : 1) of PC to Eudragit®, the stability of the formulation could be increased and the medication showed lower side effects such as flatulence as the known PF-formulations.

## Claims

1. Pharmaceutical formulation, comprising:
at least one phosphatidylcholine product (PC) of formula (I), 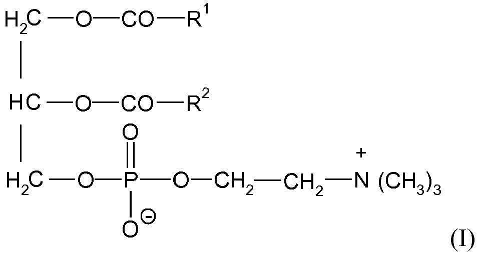
wherein:
R¹ is the residue of a saturated or unsaturated fatty acid with 12-24 carbon atoms,
and R² is the residue of a saturated or unsaturated fatty acid with 12-24 carbon atoms, and wherein the phosphatidylcholine product (PC) contains the following amounts of fatty acids (in weight percent of the total amount of fatty acids in the PC):
| | |
|---|---|
| 55 - 72 | linoleic acid |
| 10 - 18 | palmitic acid |
| 07 - 15 | oleic acid |
| 02 - 08 | linolenic acid |
| 02 - 08 | stearic acid, |
and at least one pharmaceutically acceptable excipient for delayed release of the phosphatidylcholine product (PC),
wherein the phosphatidylcholine product (PC) denotes at least 94 % by weight of all lipid/phospholipid components of the formulation.

2. Pharmaceutical formulation according to claim 1, comprising at least one phosphatidylcholine product (PC) of formula (I), wherein:
R¹ is the residue of a saturated or unsaturated fatty acid with 14-20 carbon atoms,
and R² is the residue of a saturated or unsaturated fatty acid with 14-20 carbon atoms, and wherein the phosphatidylcholine product (PC) contains the following amounts of fatty acid residues (in weight percent of the total amount of fatty acids in the PC):
| | |
|---|---|
| 59 - 70 | linoleic acid |
| 12 - 17 | palmitic acid |
| 07 - 15 | oleic acid |
| 03 - 07 | linolenic acid |
| 02 - 05 | stearic acid |
and at least one pharmaceutically acceptable excipient, wherein the pharmaceutical formulation releases at least 50 %, in particular at least 70 % by weight of the phosphatidylcholine product (PC) in the intestine.

3. Pharmaceutical formulation according to claim 1 or claim 2, wherein the pharmaceutical formulation comprises at least one phosphatidylcholine product (PC) of formula (I), wherein more than 78 %, in particular more than 80 %, by weight of the fatty acid residues R¹ and R² are unsaturated fatty acid with 18 carbon atoms.

4. Pharmaceutical formulation according to one of the claims 1 to 3, wherein the pharmaceutical formulation comprises enteric coated pellet(s), enteric coated capsule(s), enteric coated granule(s) or enteric coated tablet(s), comprising the phosphatidylcholine product (PC) and at least one layer of an enteric polymer coating, which is resistant to gastric acids.

5. Pharmaceutical formulation according to one of the claims 1 to 4, wherein the weight ratio of enteric polymer coating and phosphatidylcholine product (PC) is from 3 : 1 to 1 : 3 and wherein the PC is in a multiple unit dosage form.

6. Pharmaceutical formulation according to one of the claims 1 to 5, comprising enteric coated pellet(s) or enteric coated capsule(s) or enteric coated granule(s) or enteric coated tablet(s), having an enteric polymer coating with a thickness of 10 to 500 micrometers.

7. Pharmaceutical formulation according to one of the claims 1 to 6, comprising the phosphatidylcholine product (PC) protected by an enteric polymer coating, which is resistant to gastric acids (pH 1) for at least 120 minutes but which allows at least 80 % of the phosphatidylcholine product (PC) to be released from the formulation at a pH of 5.5 or higher within 120 minutes.

8. Pharmaceutical formulation according to one of the claims 1 to 7, comprising at least one coating with at least one enteric polymer from the group of homo-polymers and co-polymers of acrylic acid, methacrylic acid, acrylic esters and methacrylic esters.

9. Pharmaceutical formulation according to one of the claims 1 to 8, comprising from 20 to 30 % by weight of a phosphatidylcholine product (PC) of formula (I) and 70 to 80 % by weight of excipients.

10. Pharmaceutical formulation according to one of the claims 1 to 9, having a long term stability, **characterized by** the amount of at least 95 % by weight of Assay PC (relative to nominal value) and less than 5 % by weight of Lyso-PC (relative to PC) after 36 months at refrigerated storage (5° C plus/minus 3° C).

11. Process for the preparation of a pharmaceutical formulation according to one of the claims 1 to 10, comprising the step of preparing pellet(s) or capsule(s) or granule(s) or tablet(s), containing at least one phosphatidylcholine product (PC) of formula (I), and then providing the pellet(s) or capsule(s) or granule(s) or tablet(s) with at least one layer of at least one enteric polymer coating.

12. Formulation according to one of the claims 1 to 10 for the treatment or prevention of a condition or disease from the following: ulcerative colitis, Crohn's disease, diversion colitis, infectious enteritis, infectious colitis, inflammation due to irradiation and inflammation due to chemotherapeutics or chemicals, in particular for the treatment of ulcerative colitis in 5-ASA-refractory patients.

13. Formulation according to claim 12 for the treatment or prevention of ulcerative colitis, Crohn's disease, or ulcerative colitis in 5-ASA-refractory or steroid-refractory patients.

14. Formulation according to one of the claims 12 or 13 for the treatment or prevention of ulcerative colitis in 5-ASA-refractory patients, where a dose of 0.5 to 8 g, in particular 2 to 6 g, of a phosphatidylcholine product (PC) of formula (I) per day is applied.

15. Formulation according to one of the claims 12 to 14 for the treatment or prevention of ulcerative colitis in 5-ASA-refractory patients, where the daily dose of phosphatidylcholine product (PC) of formula (I) is applied twice or three times or four times per day.

16. Formulation according to one of the claims 12 to 15 for the treatment of ulcerative colitis in 5-ASA-refractory patients, where a daily dose of 0.5 to 8 g of phosphatidylcholine product (PC) of formula (I) is applied in the form of PC-containing pellets in a single dose container.

## Patentansprüche

1. Pharmazeutische Formulierung, umfassend:
mindestens ein Phosphatidylcholin-Produkt (PC) nach Formel (I), 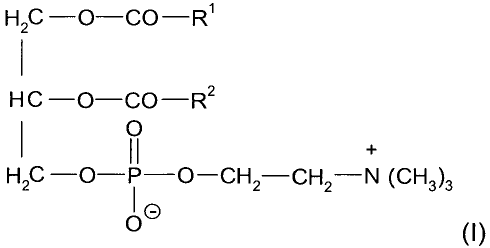
wobei:
R¹ der Rest einer gesättigten oder ungesättigten Fettsäure mit 12-24 Kohlenstoffatomen ist und R² der Rest einer gesättigten oder ungesättigten Fettsäure mit 12-24 Kohlenstoffatomen ist und wobei das Phosphatidylcholin-Produkt (PC) die folgenden Mengen an Fettsäuren (in Gewichtsprozent der Gesamtmenge an Fettsäuren in dem PC) enthält:
| | |
|---|---|
| 55 - 72 | Linolsäure |
| 10 - 18 | Palmitinsäure |
| 07 - 15 | Ölsäure |
| 02 - 08 | Linolensäure |
| 02 - 08 | Stearinsäure, |
und mindestens einen pharmazeutisch verträglichen Hilfsstoff für verzögerte Freisetzung des Phosphatidylcholin-Produkts (PC),
wobei das Phosphatidylcholin-Produkt (PC) mindestens 94 % pro Gewicht aller Lipid-/Phospholipid-Komponenten der Formulierung ausmacht.

2. Pharmazeutische Formulierung gemäß Anspruch 1, umfassend mindestens ein Phosphatidylcholin-Produkt (PC) nach Formel (I), wobei:
R¹ der Rest einer gesättigten oder ungesättigten Fettsäure mit 14-20 Kohlenstoffatomen ist und R² der Rest einer gesättigten oder ungesättigten Fettsäure mit 14-20 Kohlenstoffatomen ist und wobei das Phosphatidylcholin-Produkt (PC) die folgenden Mengen an Fettsäuren (in Gewichtsprozent der Gesamtmenge an Fettsäuren in dem PC) enthält:
| | |
|---|---|
| 59 - 70 | Linolsäure |
| 12 - 17 | Palmitinsäure |
| 07 - 15 | Ölsäure |
| 03 - 07 | Linolensäure |
| 02 - 05 | Stearinsäure, |
und mindestens einen pharmazeutisch verträglichen Hilfsstoff, wobei die pharmazeutische Formulierung mindestens 50 Gew.-%, insbesondere mindestens 70 Gew.-%, des Phosphatidylcholin-Produkts (PC) im Intestinum freisetzt.

3. Pharmazeutische Formulierung gemäß Anspruch 1 oder Anspruch 2, wobei die pharmazeutische Formulierung mindestens ein Phosphatidylcholin-Produkt (PC) nach Formel (I) umfasst, wobei mehr als 78 Gew.-%, insbesondere mehr als 80 Gew.-%, der Fettsäurereste R¹ und R² ungesättigte Fettsäuren mit 18 Kohlenstoffatomen sind.

4. Pharmazeutische Formulierung gemäß einem der Ansprüche 1 bis 3, wobei die pharmazeutische Formulierung magensaftresistente Pellet(s), magensaftresistente Kapsel(n), magensaftresistente Granulat(e) oder magensaftresistente Tablette(n) umfasst, umfassend das Phosphatidylcholin-Produkt (PC) und mindestens eine Schicht einer magensaftresistenten Polymerbeschichtung, die resistent gegenüber Magensäuren ist.

5. Pharmazeutische Formulierung gemäß einem der Ansprüche 1 bis 4, wobei das Gewichtsverhältnis von magensaftresistenter Polymerbeschichtung und Phosphatidylcholin-Produkt (PC) von 3 : 1 bis 1 : 3 ist und wobei das PC in einer multipartikulären Arzneiform vorliegt.

6. Pharmazeutische Formulierung gemäß einem der Ansprüche 1 bis 5, umfassend magensaftresistente Pellet(s), magensaftresistente Kapsel(n), magensaftresistente Granulat(e) oder magensaftresistente Tablette(n), die eine magensaftresistente Polymerbeschichtung mit einer Dicke von 10 bis 500 Mikrometern aufweisen.

7. Pharmazeutische Formulierung gemäß einem der Ansprüche 1 bis 6, umfassend das durch eine magensaftresistente Polymerbeschichtung geschützte Phosphatidylcholin-Produkt (PC), das gegenüber Magensäuren (pH 1) für mindestens 120 Minuten resistent ist, das aber mindestens 80 % des Phosphatidylcholin-Produkts (PC) erlaubt, aus der Formulierung bei einem pH von 5,5 oder höher innerhalb von 120 Minuten freigesetzt zu werden.

8. Pharmazeutische Formulierung gemäß einem der Ansprüche 1 bis 7, umfassend mindestens eine Beschichtung mit mindestens einem magensaftresistenten Polymer aus der Gruppe von Homopolymeren und Copolymeren von Acrylsäure, Methacrylsäure, Acrylestern und Methacrylestern.

9. Pharmazeutische Formulierung gemäß einem der Ansprüche 1 bis 8, umfassend von 20 bis 30 Gew.-% eines Phosphatidylcholin-Produkts (PC) nach Formel (I) und 70 bis 80 Gew.-% an Hilfsstoffen.

10. Pharmazeutische Formulierung gemäß einem der Ansprüche 1 bis 9, mit einer Langzeitstabilität, charakterisiert durch eine Menge von mindestens 95 Gew.-% von Assay-PC (relativ zum Nominalwert) und weniger als 5 Gew.-% von Lyso-PC (relativ zu PC) nach 36 Monaten bei gekühlter Lagerung (5°C plus/minus 3°C).

11. Verfahren zur Herstellung einer pharmazeutischen Formulierung gemäß einem der Ansprüche 1 bis 10, umfassend die Schritte des Herstellens von Pellet(s) oder Kapsel(n) oder Granulat(en) oder Tablette(n), enthaltend mindestens ein Phosphatidylcholin-Produkt (PC) nach Formel (I), und dann Versehen der Pellet(s) oder Kapsel(n) oder Granulat(en) oder Tablette(n) mit mindestens einer Schicht von mindestens einer magensaftresistenen Polymerbeschichtung.

12. Formulierung gemäß einem der Ansprüche 1 bis 10 zur Behandlung oder Vorbeugung eines Zustands oder einer Krankheit des Folgenden: Colitis ulcerosa, Morbus Crohn, Diversions-Colitis, infektiöse Darmentzündung, infektiöse Colitis, Entzündung durch Bestrahlung und Entzündung durch Chemotherapeutika oder Chemikalien, insbesondere zur Behandlung von Colitis ulcerosa in 5-ASA-refraktorischen Patienten.

13. Formulierung gemäß Anspruch 12 zur Behandlung oder Vorbeugung von Colitis ulcerosa, Morbus Crohn oder Colitis ulcerosa in 5-ASArefraktorischen oder Steroid-refraktorischen Patienten.

14. Formulierung gemäß einem der Ansprüche 12 oder 13 zur Behandlung oder Vorbeugung von Colitis ulcerosa in 5-ASA-refraktorischen Patienten, wobei eine Dosis von 0,5 bis 8 g, insbesondere 2 bis 6 g, eines Phosphatidylcholin-Produkts (PC) nach Formel (I) pro Tag verabreicht wird.

15. Formulierung gemäß einem der Ansprüche 12 bis 14 zur Behandlung oder Vorbeugung von Colitis ulcerosa in 5-ASA-refraktorischen Patienten, wobei die tägliche Dosis an Phosphatidylcholin-Produkt (PC) nach Formel (I) zweioder dreimal oder viermal pro Tag verabreicht wird.

16. Formulierung gemäß einem der Ansprüche 12 bis 15 zur Behandlung von Colitis ulcerosa in 5-ASA-refraktorischen Patienten, wobei eine Dosis von 0,5 bis 8 g an Phosphatidylcholin-Produkt (PC) nach Formel (I) in Form eines PC-enthaltenen Pellets in einem Einzeldosisbehältnis verabreicht wird.

## Revendications

1. Formule pharmaceutique, comprenant:
au moins un produit de phosphatidylcholine (PC) de formule (I), 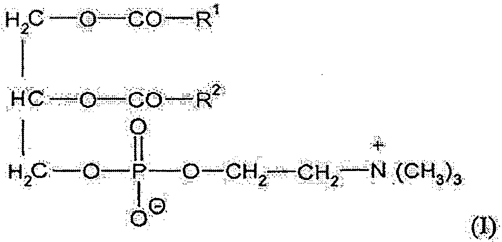
où :
R¹ est le résidu d'un acide gras saturé ou insaturé comportant 12 à 24 atomes de carbone, et R² est le résidu d'un acide gras saturé ou insaturé comportant 12 à 24 atomes de carbone, et où le produit de phosphatidylcholine (PC) contient les quantités suivantes des acides gras (en pourcentage massique de la quantité totale d'acides gras dans le PC) :
| | |
|---|---|
| 55 - 72 | acide linoléique |
| 10 - 18 | acide palmitique |
| 07 - 15 | acide oléique |
| 02 - 08 | acide linolénique |
| 02 - 08 | acide stéarique, |
et au moins un excipient pharmaceutiquement acceptable pour la libération retardée du produit de phosphatidylcholine (PC),
où le produit de phosphatidylcholine (PC) représente au moins 94 % en masse de tous les composants lipidiques/phospholipidiques de la formule.

2. Formule pharmaceutique selon la revendication 1, comprenant au moins un produit de phosphatidylcholine (PC) de formule (I), où :
R¹ est le résidu d'un acide gras saturé ou insaturé comportant 14 à 20 atomes de carbone, et R² est le résidu d'un acide gras saturé ou insaturé comportant 14 à 20 atomes de carbone, et où le produit de phosphatidylcholine (PC) contient les quantités suivantes des résidus d'acides gras (en pourcentage massique de la quantité totale d'acides gras dans le PC) :
| | |
|---|---|
| 59 - 70 | acide linoléique |
| 12 - 17 | acide palmitique |
| 07 - 15 | acide oléique |
| 03 - 07 | acide linolénique |
| 02 - 05 | acide stéarique |
et au moins un excipient pharmaceutiquement acceptable, où la formule pharmaceutique libère au moins 50 %, en particulier au moins 70 % en masse du produit de phosphatidylcholine (PC) dans l'intestin.

3. Formule pharmaceutique selon la revendication 1 ou la revendication 2, où la formule pharmaceutique comprend au moins un produit de phosphatidylcholine (PC) de formule (I), où plus de 78 %, en particulier plus de 80 %, en masse des résidus d'acides gras R¹ et R² sont des acides gras insaturés comportant 18 atomes de carbone.

4. Formule pharmaceutique selon l'une des revendications 1 à 3, où la formule pharmaceutique comprend une ou plusieurs billes à enrobage entériques, capsules à enrobage entérique, granules à enrobage entérique ou comprimés à enrobage entérique, comprenant le produit de phosphatidylcholine (PC) et au moins une couche d'un enrobage polymère entérique, qui est résistant aux acides gastriques.

5. Formule pharmaceutique selon l'une des revendications 1 à 4, où le rapport massique de l'enrobage du polymère entérique sur le produit de phosphatidylcholine (PC) est compris entre 3:1 et 1:3, et où le PC est sous forme galénique unitaire multiple.

6. Formule pharmaceutique selon l'une des revendications 1 à 5, comprenant une ou plusieurs billes à enrobage entérique, capsules à enrobage entérique, granules à enrobage entérique ou comprimés à enrobage entérique, présentant un enrobage polymère entérique d'une épaisseur de 10 à 500 micromètres.

7. Formule pharmaceutique selon l'une des revendications 1 à 6, comprenant le produit de phosphatidylcholine (PC) protégé par un enrobage polymère entérique, qui est résistant aux acides gastriques (pH 1) pendant au moins 120 minutes, mais qui permet à au moins 80 % du produit de phosphatidylcholine (PC) d'être libérés par la formule à un pH de 5,5 ou plus dans un délai de 120 minutes.

8. Formule pharmaceutique selon l'une des revendications 1 à 7, comprenant au moins un revêtement avec au moins un polymère entérique choisi dans le groupe des homopolymères et des copolymères d'acide acrylique, d'acide méthacrylique, d'esters acryliques et d'esters méthacryliques.

9. Formule pharmaceutique selon l'une des revendications 1 à 8, comprenant entre 20 et 30 % en masse d'un produit de phosphatidylcholine (PC) de formule (I) et 70 à 80 % en masse d'excipients.

10. Formule pharmaceutique selon l'une des revendications 1 à 9, présentant une stabilité à long terme, **caractérisée par** la quantité d'au moins 95 % en masse de PC de dosage (par rapport à une valeur nominale) et de moins de 5 % en masse de Lyso-PC (par rapport à PC) après 36 mois en stockage réfrigéré (5 °C plus ou moins 3 °C).

11. Procédé de préparation d'une formule pharmaceutique selon l'une des revendications 1 à 10, comprenant l'étape de préparation d'une ou plusieurs billes, capsules, granules ou comprimés, contenant au moins un produit de phosphatidylcholine (PC) de formule (I), puis d'appliquer à la ou aux billes, capsules, granules ou comprimés au moins une couche d'au moins un enrobage polymère entérique.

12. Formule selon l'une des revendications 1 à 10 pour le traitement prophylactique ou thérapeutique d'un état pathologique ou d'une maladie choisis parmi les suivants : rectocolite hémorragique, maladie de Crohn, colite de diversion, entérique infectieuse, colite infectieuse, inflammation due à une irradiation et inflammation due à des produits chimiothérapiques ou chimiques, en particulier dans le traitement de la rectocolite hémorragique chez des patients réfractaires à 5-ASA.

13. Formule selon la revendication 12 pour le traitement prophylactique ou thérapeutique de la rectocolite hémorragique, de la maladie de Crohn ou de la rectocolite hémorragique chez des patients réfractaires à 5-ASA ou aux stéroïdes.

14. Formule selon l'une des revendications 12 ou 13 pour le traitement prophylactique ou thérapeutique de la rectocolite hémorragique chez des patients réfractaires à 5-ASA, où une dose de 0,5 à 8 g, en particulier de 2 à 6 g, d'un produit de phosphatidylcholine (PC) de formule (I) est appliquée.

15. Formule selon l'une des revendications 12 à 14 pour le traitement prophylactique ou thérapeutique de la rectocolite hémorragique chez des patients réfractaires à 5-ASA, où la dose quotidienne du produit de phosphatidylcholine (PC) de formule (I) est appliquée deux ou trois fois ou quatre fois par jour.

16. Formule selon l'une des revendications 12 à 15 pour le traitement thérapeutique de la rectocolite hémorragique chez des patients réfractaires à 5-ASA, où une dose quotidienne de 0,5 à 8 g de produits de phosphatidylcholine (PC) de formule (I) est appliquée sous la forme de billes contenant le PC dans un récipient de dose unique.
